# EUROPÄISCHE PATENTSCHRIFT

(11) **EP 3 409 221 B1**
(45) Veröffentlichungstag und Bekanntmachung des Hinweises auf die Patenterteilung: **05.08.2020**
(21) Anmeldenummer: 18174531.6
(22) Anmeldetag: 28.05.2018
(51) Int. Cl.: A61B 17/34, A61B 90/00

(54) **INSTRUMENTENSYSTEM FÜR DIE MINIMALINVASIVE CHIRURGIE IM GEWEBE EINES PATIENTEN**
INSTRUMENT SYSTEM FOR MINIMALLY INVASIVE SURGERY IN THE TISSUE OF A PATIENT
SYSTÈME D'INSTRUMENT POUR CHIRURGIE À INVASION MINIMALE DANS LE TISSU D'UN PATIENT

(30) Priorität: 30.05.2017 DE 102017111821
(43) Veröffentlichungstag der Anmeldung: 05.12.2018
(73) Patentinhaber: Gaiselmann, Thomas, 78667 Villingendorf (DE); Bachmann, Karl-Heinz, 78667 Villingendorf (DE); Riek, Siegfried, 78628 Rottweil (DE)
(72) Erfinder: Gaiselmann, Thomas, 78667 Villingendorf (DE); Bachmann, Karl-Heinz, 78667 Villingendorf (DE); Riek, Siegfried, 78628 Rottweil (DE)
(74) Vertreter: Westphal, Mussgnug & Partner Patentanwälte mbB

(56) Entgegenhaltungen:
- WO-A1-95/10982
- DE-A1-102011 107 615
- US-A- 4 924 851
- US-A- 6 007 483
- US-A1- 2009 048 622
- US-A1- 2009 192 352

## Beschreibung

Die Erfindung betrifft ein Instrumentensystem für die minimalinvasive Chirurgie im Gewebe eines Patienten gemäß dem Oberbegriff des Patentanspruchs 1.

Bei der minimalinvasiven Chirurgie in natürlichen Hohlräumen des Körpers, z.B. im Bauchraum, werden Trokare verwendet, die aus einem Trokardorn und einer Trokarhülse bestehen. Mittels des Trokardorns wird das die Körperhöhle umschließende Gewebe durchstoßen, um die Trokarhülse einzusetzen. In den Körperhohlraum wird Gas insuffliert, um den Hohlraum aufzuweiten und ein ausreichendes Operationsfeld zu schaffen. Der Trokardorn wird dann herausgezogen und die Trokarhülse verbleibt als Zugang zu dem Operationsfeld für eine endoskopische Optik und für Operationsinstrumente.

Bei der minimalinvasiven Chirurgie im Gewebe eines Patienten, z. B. bei dem operativen Entfernen eines im Gewebe eingebetteten Tumors besteht eine Schwierigkeit darin, dass kein natürlicher Hohlraum für den operativen Eingriff vorhanden ist. Die US 2015/0051495 A1 beschreibt daher ein Verfahren für die minimalinvasive Chirurgie im Gewebe eines Patienten, z. B. zur Entfernung eines Tumors in der weiblichen Brust, bei welchem ein Trokar mit einem optischen Obturator unter Sicht in das Gewebe eingestochen wird, bis die distale Spitze des Trokars an den zu präparierenden Tumor gelangt. Anschließend wird durch den Trokar Gas unter Druck insuffliert, um das Gewebe auseinander zu drücken und einen künstlichen Hohlraum für die durchzuführende Operation zu schaffen. Durch Arbeitskanäle der Trokarhülse können Instrumente eingeführt werden, um die Operationsschritte vor der distalen Spitze des Trokar auszuführen.

Ein Trokar mit einem Trokardorn, einer Arbeitskanäle aufweisenden Trokarhülse und einer endoskopischen Optik ist aus der DE 10 2011 107 615 A1 bekannt.

Der Erfindung liegt die Aufgabe zugrunde, ein Instrumentensystem für die minimalinvasive Chirurgie im Gewebe eines Patienten zur Verfügung zu stellen, welches die Dissektion eines artifiziellen Operationshohlraumes für den chirurgischen Eingriff ermöglicht.

Diese Aufgabe wird erfindungsgemäß gelöst durch ein Instrumentensystem mit den Merkmalen des Patentanspruchs 1.

Vorteilhafte Ausführungen der Erfindung sind in den Unteransprüchen angegeben.

Das erfindungsgemäße Instrumentensystem weist einen langgesteckten formstabilen Instrumentenschaft auf, der in den Körper des Patienten eingeführt wird. Das distale Ende des Instrumentenschaftes gelangt dabei intrakorporal in das Zielgebiet im Gewebe, während das proximale Ende des Instrumentenschaftes extrakorporal verbleibt. Der Instrumentenschaft weist einen axial vom proximalen zum distalen Ende geradlinig durchgehenden Innenkanal auf. In diesen Innenkanal kann ein Obturator eingeschoben werden, der eine distale transparente Spitze aufweist und eine Optik aufnehmen kann, mittels welcher bei der Penetration des Gewebes das an der transparenten Spitze anliegende Gewebe visualisiert werden kann, um das Eindringen und Positionieren der distalen Spitze beobachten zu können. Weiter weist der Instrumentenschaft wenigstens einen Arbeitskanal auf, der ebenfalls axial vom proximalen zum distalen Ende durchgehend ausgebildet ist. Durch die Arbeitskanäle können semiflexible Instrumente in der Weise eingeführt werden, dass ihre distalen Wirkelemente distal aus dem Arbeitskanal austreten und vor der distalen Spitze des Instrumentenschaftes eingesetzt werden können, während die am proximalen Ende des Instruments angeordneten Betätigungselemente extrakorporal verbleiben. Weiter kann durch einen Arbeitskanal Gas unter Druck insuffliert werden, um einen künstlichen Hohlraum für die durchzuführende Operation im Gewebe zu schaffen. Ebenso kann erforderlichenfalls über einen Arbeitskanal Spülflüssigkeit zugeführt werden und/oder Blut und Gewebeflüssigkeit abgesaugt werden.

Am proximalen Ende des Innenkanals ist ein Ventilblock an dem Instrumentenschaft angeordnet. Der Ventilblock verschließt den Innenkanal abdichtend, wenn kein Instrument in den Innenkanal eingeführt ist. Wird der Obturator in den Innenkanal eingeführt, so dichtet der Ventilblock den Obturator an seinem Au-ßenumfang in dem Innenkanal ab. Wird der Obturator nach dem Positionieren der distalen Spitze im Gewebe herausgezogen, so kann anstelle des Obturators eine endoskopische Optik in den Innenkanal eingeführt werden, um den Operationsvorgang unter direkter Sicht durchführen zu können. Dabei wird auch die endoskopische Optik durch den Ventilblock in dem Innenkanal abgedichtet.

Der Innenkanal verläuft geradlinig in dem Instrumentenschaft bis zu seinem proximalen Ende, so dass der im Wesentlichen starre gerade Obturator und die Optik in diesen Innenkanal eingeführt werden können. Die Arbeitskanäle sind dagegen an ihrem proximalen Endbereich, der bei der Operation extrakorporal verbleibt, von der Achse des Instrumentenschaftes nach außen abgewinkelt bzw. abgebogen, so dass sich eine ergonomisch günstige Position für die Betätigung der in die Arbeitskanäle eingesetzten Instrumente ergibt. Ein solcher abgewinkelter Verlauf der Arbeitskanäle ist möglich, da semiflexible Instrumente verwendet werden. Auch an den proximalen Enden der Arbeitskanäle sind jeweils Ventile angeordnet, die das abgedichtete Einführen der Instrumente ermöglichen und die Arbeitskanäle abgedichtet verschließen, wenn keine Instrumente in den Arbeitskanal eingeführt sind. Ebenso dienen diese Ventile für die Insufflation des Gases unter Druck.

Weiter sind am proximalen Ende des Instrumentenschaftes vorzugsweise zwei Handgriffe angeformt, die das gesteuerte Verschieben und Bewegen des Instrumentenschaftes durch den Operateur begünstigen. Es ist vorteilhaft, wenn die proximalen Enden der Arbeitskanäle räumlich so zueinander angeordnet sind, dass der Operateur mit derselben Hand sowohl einen Handgriff halten als auch ein in dem entsprechenden Arbeitskanal angeordnetes Instrument betätigen kann.

Das distale Ende des Instrumentenschaftes ist in der Weise abgeschrägt, dass die Ebene der distalen Endstirnfläche des Instrumentenschaftes unter einem von 90° verschiedenen Winkel zur Achse des Instrumentenschaftes verläuft.

Ist der Obturator in den Instrumentenschaft eingesetzt, so ragt seine penetrierende distale Spitze über die distale Endstirnfläche des Instrumentenschaftes hinaus, um ein Penetrieren des Gewebes zu ermöglichen. Dabei sitzt das distale Ende des Obturators mit seinem Außenumfang formdicht in dem Innendurchmesser des Innenkanals, um ein Eindringen von Gewebe und dergleichen in den Innenkanal beim Vordringen der distalen Spitze zu vermeiden.

Die Endstirnfläche des Instrumentenschaftes geht dabei vorzugsweise stufenlos in die Umfangsmantelfläche der Spitze des Obturators über, sodass das von der Spitze des Obturators penetrierte Gewebe durch das sich anschließende distale Ende des Instrumentenschaftes stumpf weiter disseziert werden kann.

Die axiale Position des Obturators in dem Instrumentenschaft kann durch geeignete Fixiermittel exakt eingehalten werden. Sobald die distale Spitze in dem Operationszielgebiet positioniert ist, wird der Obturator herausgezogen. Die Optik wird in den Innenkanal eingeführt, bis ihr distales Ende am distalen Austritt des Innenkanals angeordnet ist. Die Optik erlaubt dabei einen direkten Blick in das Operationsfeld. Die Optik kann in ihrer Position dabei ebenfalls in dem Instrumentenschaft mit einer Klemmung für die Optik fixiert werden. Da eine Penetration des Gewebes in diesem Stadium nicht mehr erfolgt, muss das distale Ende der Optik nicht abgedichtet in dem Innenkanal sitzen. Es ist daher möglich, dieselbe endoskopische Optik, die in den Obturator eingesetzt wird, auch ohne den Obturator für die Beobachtung des Opertionsfeldes zu verwenden. Um ein Eindringen von Gewebe, Flüssigkeit und dergleichen in die distal offenen Arbeitskanäle während der Penetration zu verhindern, können die Arbeitskanäle gegebenenfalls durch eine jeweils eingesetzten Mandrin verschlossen werden.

Beim Eindringen in das Gewebe wird dieses durch die distal überstehende Spitze des Obturators penetriert, während das abgeschrägte distale Ende des Instrumentenschaftes das penetrierte Gewebe stumpf disseziert. Sobald die distale Spitze im Operationszielgebiet angelangt ist, kann der Instrumentenschaft um seine Längsachse rotiert werden, wodurch aufgrund der abgeschrägten Spitze des Instrumentenschaftes und der exzentrischen Anordnung der Obturatorspitze ein Ringraum im Gewebe disseziert wird, der das Insufflieren von Gas unter Druck begünstigt.

Der Instrumentenschaft ist vorzugsweise als einstückiges Kunststoffteil hergestellt.

Im Folgenden wird die Erfindung anhand eines in der Zeichnung dargestellten Ausführungsbeispiels näher erläutert. Es zeigen:

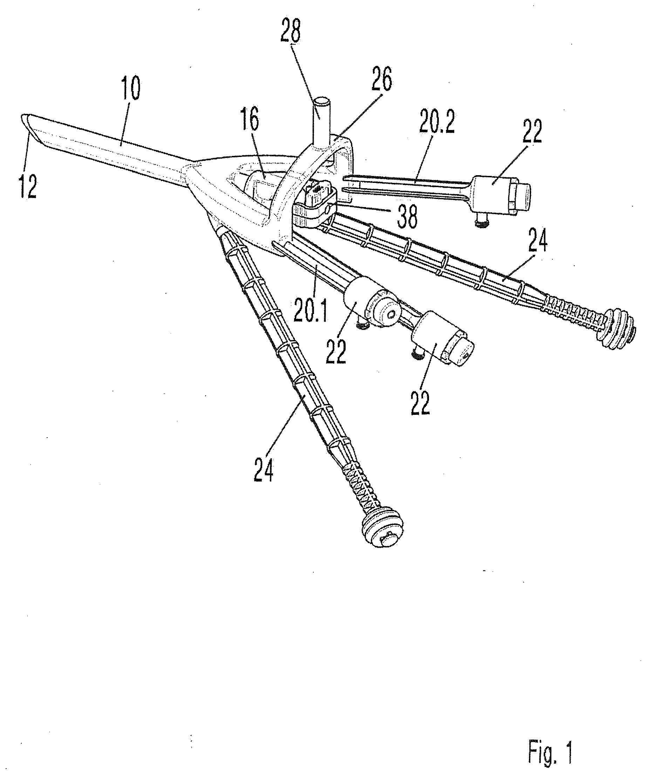

- Figur 1: in perspektivischer Ansicht ein Instrumentensystem gemäß der Erfindung,

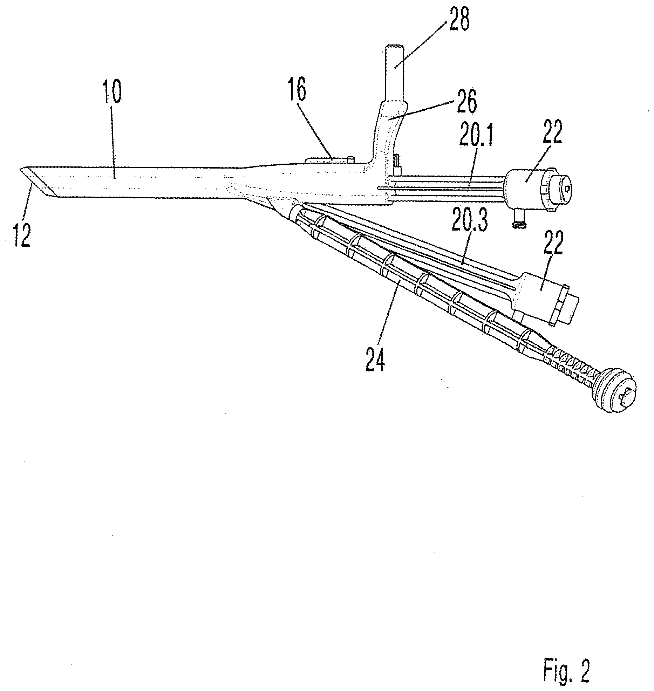

- Figur 2: einen Seitenansicht des Instrumentensystems,
- Figur 3: eine Draufsicht auf das Instrumentensystem von oben,

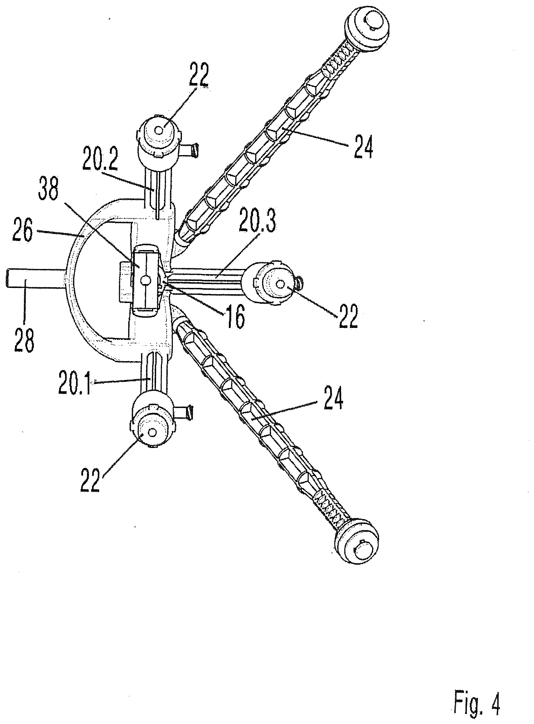

- Figur 4: eine Axialsicht auf das proximale Ende des Instrumentensystems,
- Figur 5: eine Axialsicht auf das distale Ende des Instrumentensystems,
- Figur 6: das Instrumentensystem mit einem Obturator,
- Figur 7: eine Figur 3 entsprechende Draufsicht auf das Instrumentensystem mit eingesetztem Obturator,

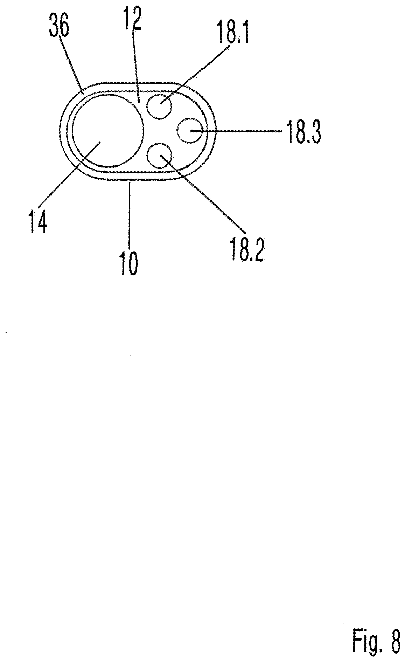

- Figur 8: eine axiale Sicht auf die distale Endstirnfläche des Instrumentenschaftes und
- Figur 9: einen Axialschnitt des distalen Endes des Instrumentensystems mit eingesetztem Obturator.

In dem dargestellten Ausführungsbeispiel weist das Instrumentensystem einen Instrumentenschaft 10 auf, der als langgestreckter gerader Zylinder mit etwa ovalem Querschnitt ausgebildet ist. Die distale vordere Endstirnfläche 12 des Instrumentenschaftes 10 ist in der Weise abgeschrägt, dass ihre Ebene mit der Achse des Instrumentenschaftes 10 einen von 90° abweichenden Winkel einschließt, der vorzugsweise zwischen 30° und 60° liegt und im dargestellten Ausführungsbeispiel 45° beträgt.

Durch den Instrumentenschaft 10 führt in dessen axialer Längsrichtung ein Innenkanal 14 hindurch, der vorzugsweise kreisförmigen Querschnitt aufweist und geradlinig vom proximalen Ende zum distalen Ende des Instrumentenschaftes 10 hindurchführt. Der Innenkanal 14 verläuft im Querschnitt des ovalen Instrumentenschaftes 10 vorzugsweise exzentrisch zu der Mittelachse des Instrumentenschaftes 10. Dadurch tritt der Innenkanal 14 am distalen Ende in dem weiter distalwärts liegenden Flächenbereich der abgeschrägten Endstirnfläche aus. Am proximalen Ende des Innenkanals 14 ist ein Ventilblock 16 angebracht. Der Ventilblock 16 weist vorzugsweise eine innere Ventilklappe und eine Lippendichtung auf. Die Ventilklappe verschließt den Innenkanal 14 luftdicht und kann geöffnet werden, um den Durchtritt eines Instruments zu ermöglichen. Wird ein Instrument in den Innenkanal 14 eingeführt und die Ventilklappe geöffnet, so legt sich die Lippendichtung am Umfang des eingeführten Instruments an und dichtet dadurch den Innenkanal 14 ab.

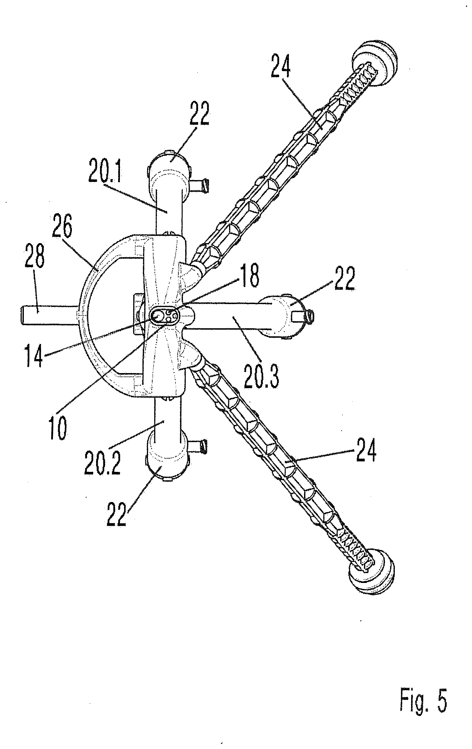

Weiter ist in dem Instrumentenschaft 10 wenigstens ein Arbeitskanal 18 ausgebildet. In dem dargestellten Ausführungsbeispiel sind drei Arbeitskanäle 18 vorgesehen. Die Arbeitskanäle 18 verlaufen achsparallel neben dem Innenkanal 14 in dem Instrumentenschaft 10 und öffnen sich distal in der Endstirnfläche 12 in deren weiter proximalwärts liegendem Flächenbereich. Wie in Figur 5 zu sehen ist, sind die drei Arbeitskanäle 18 im Querschnitt des Instrumtenschaftes 10 dreieckig unter dem Innenkanal 14 angeordnet.

Die Arbeitskanäle 18 verlaufen in dem Instrumentenschaft 10 achsparallel geradlinig bis zu dem proximalen Ende des Instrumentenschaftes 10. Am proximalen Ende des Instrumtenschaftes 10 verlaufen die Arbeitskanäle 18 gegen die Längsachse des Instrumentenschaftes 10 nach außen abgewinkelt, so dass sie mit der Achse des Instrumentenschaftes 10 einen sich proximalwärts öffnenden Winkel einschließen, der vorzugsweise zwischen 15° und 45° liegt, im dargestellten Ausführungsbeispiel etwa 30° beträgt. Der abgewinkelte proximale Endbereich der Arbeitskanäle 18 wird jeweils durch einen an das proximale Ende des Instrumentenschaftes 10 einstückig angeformten rohrförmigen Ansatz 20 gebildet. Die rohrförmigen Ansätze 20, die den Arbeitskanal 18 einschließen, erstrecken sich proximalwärts über das proximale Ende des Instrumentenschaftes 10 und den Ventilblock 16 hinaus. Im dargestellten Ausführungsbeispiel sind zwei Ansätze 20.1 und 20.2 mit den jeweiligen Arbeitskanälen 18.1 und 18.2 in einer Ebene angeordnet, die die Achse des Instrumentenschaftes 10 einschließt. Der dritte Ansatz 20.3 mit dem Arbeitskanal 18.3 ist von dieser Ebene nach unten abgewinkelt.

Am proximalen Ende der Ansätze 20 ist jeweils ein Ventil 22 angeordnet, welches den jeweiligen Arbeitskanal 18 abdichtend abschließt und ein abgedichtetes Einführen eines Instruments ermöglicht, wie dies vorstehend für den Ventilblock 16 erläutert ist.

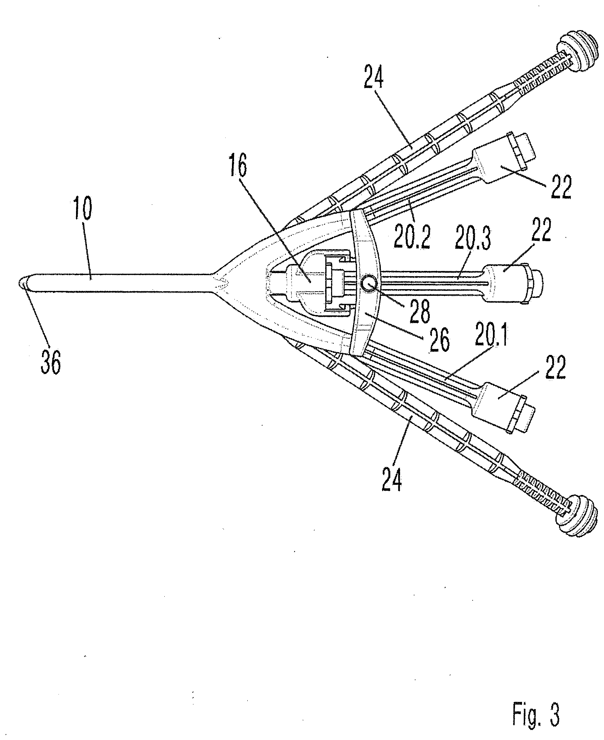

Weiter ist am proximalen Ende des Instrumentenschaftes 10 wenigstes ein Handgriff 24 angeformt. Im dargestellten Ausführungsbeispiel sind zwei Handgriffe 24 vorhanden. Die Handgriffe 24 sind als einstückig angeformte Stäbe ausgebildet, die gegen die Achse des Instrumentenschaftes 10 abgewinkelt sind und mit der Achse des Instrumentenschaftes 10 einen sich proximalwärts öffnenden Winkel von etwa 15° bis 45°, vorzugsweise von etwa 30° einschließen. Wie insbesondere aus Figur 3 zu erkennen ist, liegen die zwei Handgriffe 24 im Wesentlichen unterhalb der Ansätze 20.1 und 20.2.

Am proximalen Ende des Instrumentenschaftes 10 ist weiter einstückig ein Bügel 26 angeformt, der sich vorzugsweise von den Ansätzen 20.1 und 20.2 ausgehend bogenförmig auswölbt. An dem Bügel 26 ist ein Stutzen 28 angeformt, der zur Befestigung des Instrumentenschaftes 10 an einem Stativ oder dergleichen dient.

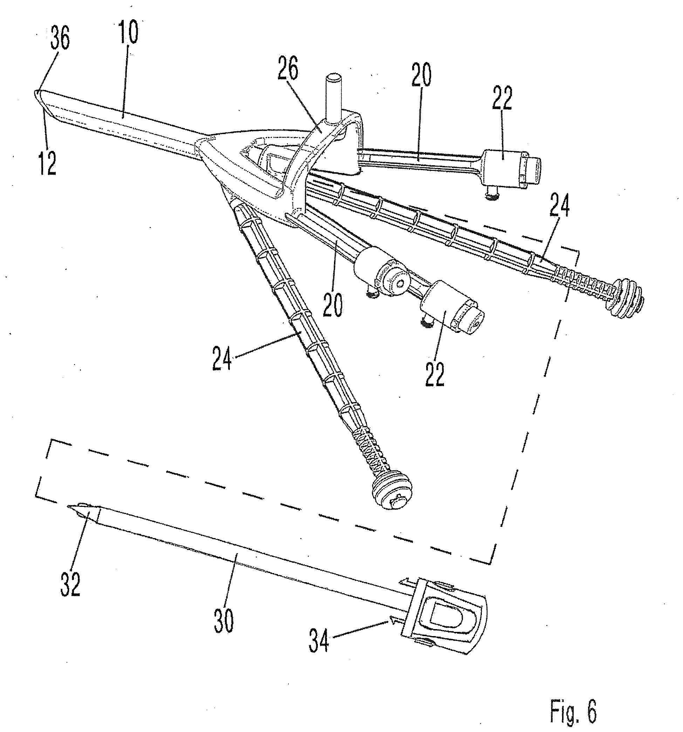

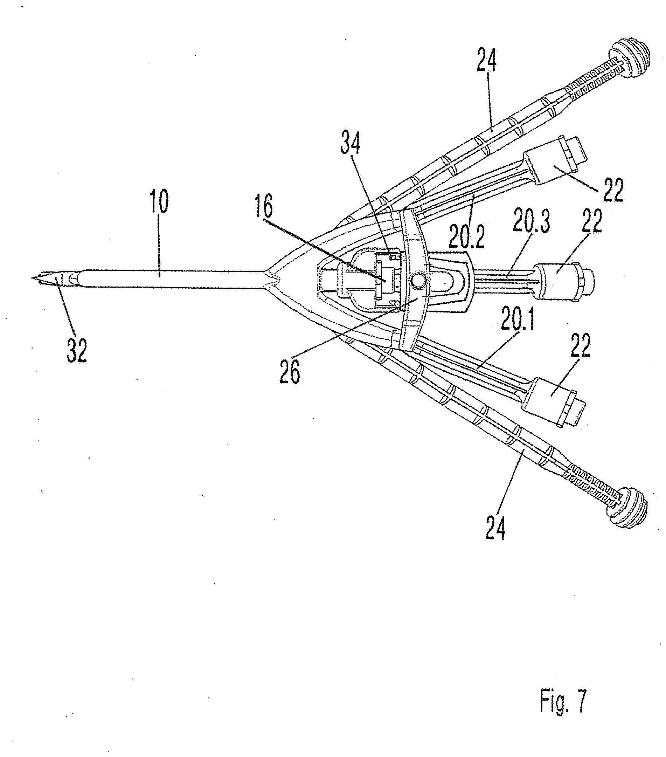

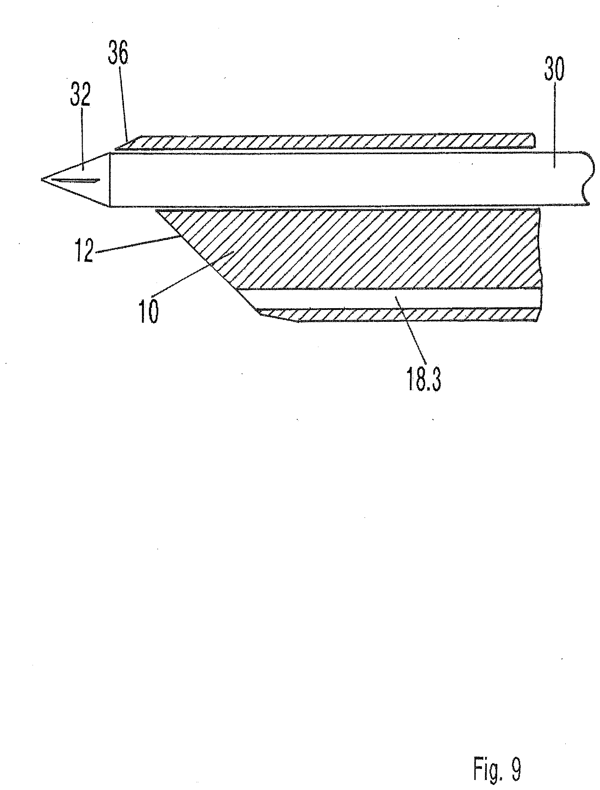

Zu dem Instrumentensystem gehört weiter ein Obturator 30. Der Obturator 30 ist als Hohlnadel mit einer transparenten distalen Spitze 32 ausgebildet. Die Spitze 32 kann scharf oder stumpf konisch ausgebildet sein. In den Obturator 30 wird eine endoskopische Optik eingeschoben. Die Optik ist in an sich bekannter Weise ausgebildet und daher in der Zeichnung nicht dargestellt und nicht näher beschrieben. Wie in den Figuren 6 und 7 dargestellt ist, wird der Obturator 30 durch den Ventilblock 16 in den Innenkanal 14 des Instrumentenschaftes 10 eingeschoben. Durch geeignete Fixiermittel 34 wird der in den Innenkanal 14 eingeführte Obturator 30 an den Ventilblock fixiert, wodurch seine axialen Position in dem Instrumentenschaft 10 definiert und festgehalten wird. Die distale Spitze 32 des Obturators 30 ragt in dieser Position distal über die Endstirnfläche 12 des Instrumentenschaftes 10 hinaus, wie Figur 9 zeigt. Der Außendurchmesser des Obturators 30 ist so auf den Innendurchmesser des Innenkanals 14 abgestimmt, dass der Obturator 30 an der Endstirnfläche 12 formdicht in dem Innenkanal 14 sitzt. Dadurch ist gewährleistet, dass Gewebe nicht in den Innenraum des Innenkanals 14 eindringen können. Bei eingesetztem Obturator 30 geht der Außenumfang der herausragenden Spitze 32 stufenlos in die Endstirnfläche 12 über, wie dies Figur 9 zeigt. Dies ergibt sich im proximalwärts liegenden Bereich (in Figur 9 unten) durch die Schrägstellung der Endstirnfläche 12. Im distalwärts liegenden Bereich (in Figur 9 oben) ist die Außenkante der Endstirnfläche 12 zwischen der Austrittsöffnung des Innenkanals 14 und dem Umfang des Instrumentenschaftes 10 durch eine Fase 36 so abgekantet, dass auch hier ein stufenloser Übergang bewirkt wird. Die Endstirnfläche 12 geht somit am gesamten Umfang der Obturatorspitze 32 in einem distalwärts spitzen Winkel von im dargestellten Beispiel 45° in den Außenumfang des Obturators 30 über.

Nach dem Entfernen des Obtruators 30 aus dem Instrumentenschaft 10 kann die endoskopische Optik aus der Hohlnadel des Obturators 30 herausgezogen werden und kann in entsprechender Weise auch durch den Ventilblock 16 in den Innenkanal 14 des Instrumentenschaftes 10 eingeschoben und in ihrer axialen Position in dem Innenschaft 10 fixiert werden. Ist die Optik allein in den Instrumentenschaft 10 eingeführt und insbesondere mit einer Klemmung 38 für die Optik fixiert, so befindet sich das distale Ende der Optik im Wesentlichen in der Ebene der Endstirnfläche 12 des Instrumentenschaftes 10.

Durch die Arbeitskanäle 18 können semiflexible Instrumente eingeführt werden. Solche semiflexiblen Instrumente sind ans sich bekannt, so dass sie hier nicht näher erläutert werden müssen. Die semiflexiblen Instrumente besitzen einen flexibel biegsamen, gegen Zug- und Druckkräfte stabilen Schaft auf. Die Instrumente können durch den jeweiligen Arbeitskanal 18 eingeführt werden, wobei sie aufgrund ihres flexiblen Schaftes der Krümmung der Arbeitskanäle 18 folgen können. Ist das Instrument in den jeweiligen Arbeitskanal 18 eingeführt, so tritt sein distales Wirkelement distal aus der Endstirnfläche 12 des Instrumentenschaftes 10 heraus. Die proximalen Betätigungselemente des Instruments verbleiben proximal außerhalb des Arbeitskanals 18 und dessen Ventil 22. Das Instrument wird durch das Ventil 22 abgedichtet. Als semiflexible Instrumente können eine Vielzahl von an sich bekannten Instrumenten mit entsprechend ausgebildeten Wirkelementen und Betätigungselementen verwendet werden. Dies können beispielsweise schneidende, klemmende, greifende, koagulierende Instrumente oder Saugspülinstrumente oder Probeentnahme-Zangen usw. sein.

Weiter kann durch die Arbeitskanäle 18 oder den Innenkanal 14 insbesondere auch Gas unter Druck insuffliert werden. Ebenso ist es möglich über die Arbeitskanäle Flüssigkeit zuzuführen und abzusaugen, z. B. zum Spülen des Operationsfeldes und zum Absaugen von Blut und Gewebeflüssigkeit.

Eine minimalinvasive Operation im Gewebe eines Patienten wird mit Hilfe des erfindungsgemäßen Instrumentensystems in folgender Weise durchgeführt:
Zunächst wird der Obturator 30 in den Instrumentenschaft 10 eingesetzt und in seiner axialen Position im Instrumentenschaft 10 so fixiert, dass die distale Spitze 32 distal aus der Endstirnfläche 12 des Instrumentenschaftes 10 herausragt. Der Instrumentenschaft 10 wird gegebenenfalls über eine Hautinzision in das Gewebe eingeführt. Dabei penetriert die distale Spitze 32 des Obturators das Gewebe. Das Vordringen des distalen Endes im Gewebe kann mittels der Optik durch die transparente Spitze 32 des Obturators 30 beobachtet werden. Das mittels der Spitze 32 penetrierte Gewebe wird durch das stufenlos anschließende distale Ende des Instrumentenschaftes 10 stumpf weiter disseziert. Da der Obturator 30 am distalen Ende formdicht im Innenkanal 14 und dem distalen Endes des Instrumentenschafts 10 sitzt, kann bei dieser Penetration des Gewebes kein Gewebe in den Innenkanal 14 eindringen. Die distalen Öffnungen der Arbeitskanäle 18 können erforderlichenfalls durch einen geeigneten Mandrin verschlossen werden, so dass auch in die Arbeitskanäle 18 kein Gewebe eindringen können.

Der Instrumentenschaft 10 wird unter Visualisierung durch die transparente Spitze 32 des Obturators 30 durch das Gewebe eingeführt, bis das distale Ende des Instrumentenschaftes 10 im Operationszielgebiet positioniert ist. Ein feinfühliges Einführen und Manövrieren des Instrumentenschaftes 10 wird dabei dem Operateur durch die Handgriffe 24 erleichtert. Ist das distale Ende des Instrumentenschaftes 10 im Operationszielgebiet positioniert, so wird der Instrumentenschaft 10 um seine Längsachse rotiert, was idealerweise durch Greifen des Instrumentes an dem Bügel 26 erleichtert wird. Bei der Rotation des Instrumentenschaftes 10 erzeugt das distale Ende des Instrumenteschaftes 10 aufgrund seiner Abschrägung und der außermittigen Anordnung des Obturators 30 einen ringförmigen Hohlraum im Gewebe. Es kann nun durch einen der Arbeitskanäle 18 oder den Innenkanal 14 Gas unter Druck in diesen anfänglich erzeugten Hohlraum insuffliert werden, wodurch das Gewebe weggedrückt und der anfänglich artifiziell geschaffene Spaltraum zu einem Hohlraum dilatiert wird. In diesen nun vor dem distalen Ende des Instrumentenschaftes 10 erzeugten künstlichen Hohlraum können nun die semiflexiblen Instrumente durch die Arbeitskanäle 18 eingeführt werden, um operative Schritte in dem Operationsgebiet durchzuführen. Hierzu wird der Obturator 30 aus dem Instrumentenschaft 10 herausgezogen, da eine weitere Penetration des Gewebes nicht mehr erforderlich ist. Anstelle des Obturators 30 wird nun eine endoskopische Optik in den Innenkanal 14 eingeführt und in diesem Innenkanal 14 positioniert und mit der Klemmung 38 für die Optik fixiert. Vorzugsweise kann hierzu die zuvor in dem Obturator 30 angeordnete Optik verwendet werden. Die eingesetzte und positionierte Optik ermöglicht eine direkte Sicht in den durch die Gasinsufflation erzeugten artifiziellen Hohlraum, so dass die operativen Schritte unter ungestörter direkter Sicht durch die Optik durchgeführt werden können. Somit entsteht ein Operationssitus wie z. B. bei einer laparokopischen Operation in einer präformierten Körperhöhle.

Die gegenseitige Anordnung der Ansätze 20 und der Handgriffe 24 ermöglicht dabei eine ergonomisch vorteilhafte Handhabung des Instrumentensystems durch den Operateur. Der Operateur kann jeweils mit einer Hand einen der Handgriffe 24 erfassen und mittels des Ringfingers und des kleinen Fingers halten, während er gleichzeitig mit Daumen, Ringfinger und Mittelfinger derselben Hand die Betätigungselemente eines Instruments in dem benachbarten Ansatz 20.1 bzw. 20.2 bedienen kann. Mittels des Bügels 26 und des Stutzens 28 kann das Instrumentensystem außerdem an einem geeigneten Stativ gehalten werden, um den Instrumentenschaft 10 während der Operation in seiner jeweiligen Position zu fixieren.

### Bezugszeichenliste

- 10: Instrumentenschaft
- 12: Endstirnfläche
- 14: Innenkanal
- 16: Ventilblock
- 18: Arbeitskanal
- 20: Ansatz
- 22: Ventil
- 24: Handgriff
- 26: Bügel
- 28: Stutzen
- 30: Obturator
- 32: Spitze
- 34: Fixiermittel
- 36: Fase
- 38: Klemmung für die Optik

## Patentansprüche

1. Instrumentensystem für die mininmalinvasive Chirurgie im Gewebe eines Patienten mit einem Instrumentenschaft (10), der ein in den Körper des Patienten einsetzbares distales Ende und ein extrakorporal verbleibendes proximales Ende aufweist, mit einem in dem Instrumentenschaft (10) axial durchgehend verlaufenden Innenkanal (14), mit wenigstens einem in dem Instrumentenschaft (10) axial durchgehend verlaufenden Arbeitskanal (18) und mit einem Obturator (30), der koaxial eine Optik aufnehmen kann und in den Innenkanal (14) in der Weise einsetzbar ist, dass seine transparente distale Spitze (32) aus der distalen Endstirnfläche (12) des Instrumentenschaftes (10) herausragt, wobei
an dem proximalen Ende des Instrumentenschaftes (10) ein Ventilblock (16) angebracht ist, der den Innenkanal (14) verschließt und einen abgedichteten Durchtritt des Obturators (30) oder einer Optik ermöglicht,
**dadurch gekennzeichnet, dass** die Ebene der distalen Endstirnfläche (12) des Instrumentenschaftes (10) in einem von 90° abweichenden Winkel gegen die Mittelachse des Instrumentenschaftes (10) abgeschrägt ist, dass der Innenkanal (14) und der wenigstens eine Arbeitskanal (18) des Instrumentenschaftes (10) in der Endstirnfläche (12) austreten und dass bei eingesetztem Obturator (30) dessen distal herausragende Spitze (32) stufenlos in die Endstirnfläche (12) übergeht.

2. Instrumentensystem nach Anspruch 1,
**dadurch gekennzeichnet, dass** die Endstirnfläche (12) des Instrumentenschaftes (10) gegen die Mittelachse des Instrumentenschaftes (10) in einem Winkel zwischen 30° und 60°, vorzugsweise in einem Winkel von etwa 45° abgeschrägt ist.

3. Instrumentensystem nach Anspruch 1 oder 2,
**dadurch gekennzeichnet, dass** der Innenkanal (14) exzentrisch zur Mittelachse des Instrumentenschafts (10) in dem distalwärts liegenden Flächenbereich der Endstirnfläche austritt.

4. Instrumentensystem nach Anspruch 1 oder 2 oder 3,
**dadurch gekennzeichnet, dass** die distalwärts liegende Außenkante der Endstirnfläche (12) zwischen der Austrittsöffnung des Innenkanals (14) und dem Außenumfang der Endstirnfläche (12) mit einer Fase (36) so abgekantet ist, dass die Endstirnfläche (12) im Bereich dieser Fase (36) in einem spitzen Winkel in den Außenumfang der aus der Endstirnfläche (12) herausragenden distalen Spitze (32) des Obturators (30) übergeht.

5. Instrumentensystem nach Anspruch 1,
**dadurch gekennzeichnet, dass** der wenigstens eine Arbeitskanal (18) in seinem proximalen Endbereich gegen die Längsachse des Instrumentenschaftes (10) in einem sich proximalwärts öffnenden Winkel abgewinkelt in einem am proximalen Ende des Instrumentenschaftes (10) angeformten Ansatz (20) verläuft und dass am proximalen Ende des Instrumentenschaftes (10) wenigstens ein Handgriff (24) angeordnet ist.

6. Instrumentensystem nach Anspruch 5,
**dadurch gekennzeichnet, dass** in dem Instrumentenschaft (10) drei Arbeitskanäle angeordnet sind, die achsparallel zu dem Innenkanal (14) auf derselben Seite neben dem Innenkanal (14) verlaufen und distal in dem proximalwärts liegenden Bereich der Endstirnfläche (12) austreten, und dass die drei Arbeitskanäle (18) in ihrem proximalen Endbereich jeweils in Ansätzen (20) verlaufen, von denen zwei Ansätze (20.1, 20.2) im Wesentlichen in einer die Längsachse des Instrumentenschaftes (10) einschließenden Ebene liegen, während der dritte Ansatz (20.3) im Winkel zu dieser Ebene verläuft.

7. Instrumentensystem nach Anspruch 5,
**dadurch gekennzeichnet, dass** zwei Handgriffe (24) am proximalen Ende des Instrumentenschafts (10) angeformt sind, die stabförmig ausgebildet sind und in einem sich proximalwärts öffnenden Winkel gegenüber der Achse des Instrumentenschaftes (10) und gegeneinander abgewinkelt sind.

8. Instrumentensystem nach den Ansprüchen 6 und 7,
**dadurch gekennzeichnet, dass** jeder der Handgriffe (24) jeweils unter einem der Ansätze (20.1 bzw. 20.2), die in einer Ebene mit der Mittelachse des Instrumentenschaftes (10) liegen, angeordnet ist.

9. Instrumentensystem nach Anspruch 5,
**dadurch gekennzeichnet, dass** jeder der Ansätze (20) der Arbeitskanäle (18) ein Ventil (22) aufweist, welches den Arbeitskanal (18) verschließt und einen abgedichteten Durchtritt eines Instruments ermöglicht.

10. Instrumentensystem nach Anspruch 1,
**dadurch gekennzeichnet, dass** die in dem Obturator (30) aufgenommene Optik aus dem Obturator (30) herausnehmbar ist und anstelle des Obturators (30) durch den Ventilblock (16) in den Innenkanal (14) des Instrumentenschaftes (10) einführbar und mit einer Klemmung (38) für die Optik reversibel fixierbar ist.

11. Instrumentensystem nach Anspruch 1,
**dadurch gekennzeichnet, dass** ein Arbeitskanal (18) oder der Innenkanal (14) als Insufflationskanal verwendbar ist.

12. Instrumentensystem nach Anspruch 1 oder 10,
**dadurch gekennzeichnet, dass** am proximalen Ende des Instrumentenschaftes (10) Fixiermittel (34) angeordnet sind, die ein axial positioniertes Fixieren des Obturators (30) ermöglichen.

13. Instrumentensystem nach Anspruch 1 oder 10,
**dadurch gekennzeichnet, dass** am proximalen Ende des Instrumentenschaftes (10) eine Klemmung (38) für die Optik angeordnet ist.

14. Instrumentensystem nach Anspruch 1,
**dadurch gekennzeichnet, dass** am proximalen Ende des Instrumentenschaftes (10) Mittel (26, 28) angeformt sind, die zur Befestigung des Instrumentenschaftes (10) an einem Operationsstativ dienen.

## Claims

1. Instrument system for minimally invasive surgery on the tissue of a patient having an instrument shaft (10) comprising a distal end which is insertable into the body of a patient and a proximal end which remains extracorporeal, an inner cannula (14) extending axially through the instrument shaft (10), at least one operating cannula (18) extending axially through the instrument shaft (10) and having an obturator (30) which can coaxially receive an optical device and which can be inserted into the inner cannula (14) so that its transparent distal tip (32) protrudes from the distal end face (12) of the instrument shaft (10),
wherein a valve block (16), is disposed on the proximal end of the instrument shaft (10), closing the inner cannula (14) and allowing for a sealed penetration of the obturator (30) or of an optical device,
**characterized in that** the plane of the distal end face (12) of the instrument shaft (10) is slanted toward the middle axis of the instrument shaft (10) at an angle different from 90°, **in that** the inner cannula (14) and the at least one operating cannula (18) of the instrument shaft (10) emerge from the distal end face (12), and **in that**, when the obturator (30) is inserted, its distal protruding tip (32) transitions gradually into the end face (12).

2. Instrument system in accordance with claim 1,
**characterized in that** the end face (12) of the instrument shaft (10) is slanted toward the middle axis of the instrument shaft (10) at an angle of between 30° and 60°, preferably at an angle of approximately 45°.

3. Instrument system in accordance with claim 1 or 2,
**characterized in that** the inner cannula (14) emerges eccentrically to the middle axis of the instrument shaft (10) in the distally disposed surface region of the end face (12).

4. Instrument system in accordance with claim 1 or 2 or 3,
**characterized in that** the distally disposed outer edge of the end face (12) is beveled between the exit opening of the inner cannula (14) and the outer periphery of the and face (12) with a chamfer (36) such that, in the region of this chamfer (36), the end face (12) transitions at an acute angle into the outer periphery of the distal tip (12) of the obturator protruding out of the end face (12).

5. Instrument system in accordance with claim 1,
**characterized in that** the at least one operating cannula (18) extends, slanted in its proximal end region toward the longitudinal axis of the instrument shaft (10) at a proximad open angle, in an extension (20) formed at the proximal end of the instruments shaft (10) and that at least one hand grip (24) is disposed at the proximal end of the instrument shaft (10).

6. Instrument system in accordance with claim 5,
**characterized in that** three operating cannulae are disposed in the instrument shaft (10) that extend paraxially to the inner cannula (14) on the same side next to the inner cannula (14) and emerge distally in the proximally disposed region of the end face (12), and **in that** each of the three operating cannulae (18) extends in its proximal end region in extensions (20), of which two extensions (20.1, 20.2) lie in a plane that essentially encompasses the longitudinal axis of the instrument shaft (10), whereas the third extension (20.3) extends at an angle to this plane.

7. Instrument system in accordance with claim 5,
**characterized in that** two hand grips (24) are formed at the proximal end of the instrument shaft (10) which are implemented in a rod-like shape and which are angled at a proximad opened angle toward the axis of the instrument shaft (10) and toward each other.

8. Instrument system in accordance with claims 6 and 7,
**characterized in that** each of the hand grips (24) is disposed underneath one of the extensions (20.1 and 20.2) that lie in a plane with the middle axis of the instrument shaft (10).

9. Instrument system in accordance with claim 5,
**characterized in that** each of the extensions (20) of the operating cannulae (18) comprises a valve (22) which closes the operating cannula (18) and allows a sealed penetration of an instrument.

10. Instrument system in accordance with claim 1,
**characterized in that** the optical device received by the obturator (30) can be taken out of the obturator (30) and can be inserted through the valve block (16) into the inner cannula (14) of the instrument shaft (10) and is reversibly securable with a clamp (38) for the optical device.

11. Instrument system in accordance with claim 1
**characterized in that** an operating cannula (18) or the inner cannula (14) can be used as an insufflation cannula.

12. Instrument system in accordance with claim 1 or 10,
**characterized in that** a securing means (34) are disposed on the proximal end of the instrument shaft (10) which allow for an axially positioned securing of the obturator (30).

13. Instrument system in accordance with claim 1 or 10,
**characterized in that** a clamp (38) for the optical device is disposed on the proximal end of the instrument shaft (10).

14. Instrument system in accordance with claim 1,
**characterized in that** means (26, 28) are formed on the proximal end of the instrument shaft (10) that serve to secure the instrument shaft (10) on an operating stand.

## Revendications

1. Système d'instruments pour une chirurgie à invasion minimale dans le tissu d'un patient comprenant une tige d'instrument (10) ayant une extrémité distale qui s'introduit dans le corps du patient et une extrémité proximale restant à l'extérieur du corps, un canal intérieur (14) traversant axialement la tige d'instrument (10), au moins un canal de travail (18) traversant axialement la tige d'instrument (10) et un obturateur (30) qui peut recevoir co-axialement une optique et se place dans le canal intérieur (14) de façon que sa pointe distale (32) transparente, dépasse de la surface frontale (12) de l'extrémité distale de la tige d'instrument (10),
dans lequel
un bloc-soupape (16) est prévu à l'extrémité proximale de la tige d'instrument (10) qui ferme le canal intérieur (14) et permet le passage étanche de l'obturateur (30) ou d'une optique,
système **caractérisé en ce que**
le plan de la surface frontale (12) de l'extrémité distale de la tige d'instrument (10) est incliné suivant un angle différent de 90° par rapport à l'axe de la tige d'instrument (10), le canal intérieur (14) et au moins un canal de travail (18) de la tige d'instrument (10) débouchent dans la surface frontale d'extrémité (12), et
lorsque l'obturateur (30) est placé, sa pointe (32) distale en saillie rejoint la surface frontale d'extrémité (12) de manière continue.

2. Système d'instruments selon la revendication 1,
**caractérisé en ce que**
la surface frontale d'extrémité (12) de la tige d'instrument (10) est inclinée par rapport à l'axe de la tige d'instrument (10) suivant un angle compris entre 30° et 60°, de préférence suivant un angle de l'ordre de 45°.

3. Système d'instruments selon la revendication 1 ou 2,
**caractérisé en ce que**
le canal intérieur (14) débouche de façon décentrée par rapport à l'axe de la tige d'instrument (10) dans la zone de la surface située côté distal de la surface frontale d'extrémité.

4. Système d'instruments selon la revendication 1 ou 2 ou 3,
**caractérisé en ce que**
l'arête extérieure de la surface frontale d'extrémité (12) côté distal, est coupée entre l'orifice de sortie du canal intérieur (14) et la périphérie extérieure de la surface frontale d'extrémité (12) avec un congé (36) de façon que la surface frontale d'extrémité (12) dans la région de ce congé (36) rejoint suivant un angle aigu, la périphérie extérieure de la pointe distale (32) de l'obturateur (30) qui dépasse de la surface frontale d'extrémité (12).

5. Système d'instruments selon la revendication 1,
**caractérisé en ce que**
dans sa zone d'extrémité proximale, au moins un canal de travail (18) est coudé par rapport à l'axe longitudinal de la tige d'instrument (10) suivant un angle ouvert, côté proximal, dans le prolongement (20) formé à l'extrémité proximale de la tige d'instrument (10) et l'extrémité proximale de la tige d'instrument (10) a au moins une poignée (24).

6. Système d'instruments selon la revendication 5,
**caractérisé en ce que**
la tige d'instrument (10) comporte trois canaux de travail, parallèle à l'axe du canal intérieur (14) du même côté, de façon adjacente au canal intérieur (14) et débouchant à l'extrémité distale dans la zone de la surface frontale d'extrémité (12) tournée vers l'extrémité proximale, et
dans leur zone d'extrémité proximale, les trois canaux de travail (18) ont chacun un prolongement (20) parmi lesquels, deux prolongements (20.1, 20.2) sont situés pratiquement dans un plan passant par l'axe longitudinal de la tige d'instrument (10) alors que le troisième prolongement (20.3) fait un angle par rapport à ce plan.

7. Système d'instruments selon la revendication 5,
**caractérisé en ce que**
deux poignées (24) sont formées à l'extrémité proximale de la tige d'instrument (10), ces poignées ont une forme de tige et sont inclinées suivant un angle ouvert, côté proximal, par rapport à l'axe de la tige d'instrument (10), en étant écarté l'un de l'autre.

8. Système d'instruments selon les revendications 6 et 7,
**caractérisé en ce que**
chacune des poignées (24) est prévue respectivement sous un prolongement (20.1 ou 20.2) qui se situe dans un plan avec l'axe de la tige d'instrument (10).

9. Système d'instruments selon la revendication 5,
**caractérisé en ce que**
chacun des prolongements (20) des canaux de travail (18) comporte une soupape (22) qui ferme le canal de travail (18) et permet le passage étanche d'un instrument.

10. Système d'instruments selon la revendication 1,
**caractérisé en ce que**
l'optique logée dans l'obturateur (30) peut être extraite de l'obturateur (30) et à la place de l'obturateur (30), s'introduire par le bloc de soupape (16) dans le canal intérieur (14) de la tige d'instrument (10) en étant fixé de manière réversible par un moyen de serrage (38) pour l'optique.

11. Système d'instruments selon la revendication 1,
**caractérisé en ce qu'**
un canal de travail (18) ou le canal intérieur (14) s'utilise comme canal d'insufflation.

12. Système d'instruments selon la revendication 1 ou 10,
**caractérisé en ce que**
des moyens de fixation (34) sont prévus à l'extrémité proximale de la tige d'instrument (10) qui permettent de fixer le positionnement axial de l'obturateur (30).

13. Système d'instruments selon la revendication 1 ou 10,
**caractérisé en ce que**
l'extrémité proximale de la tige d'instrument (10) comporte un moyen de serrage (38) pour l'optique.

14. Système d'instruments selon la revendication 1,
**caractérisé en ce que**
l'extrémité proximale de la tige d'instrument (10) comporte des moyens (26, 28) servant à la fixation de la tige d'instrument (10) à un pied d'opération.
